(19) **Europäisches Patentamt**

**European Patent Office**

**Office européen des brevets**

(11) **EP 1 005 908 B1**

(12) **EUROPÄISCHE PATENTSCHRIFT**

(45) Veröffentlichungstag und Bekanntmachung des Hinweises auf die Patenterteilung:
**20.09.2006 Patentblatt 2006/38**

(51) Int Cl.:
*B01J 37/04* (2006.01)    *B01J 37/02* (2006.01)
*B01J 23/887* (2006.01)    *C07C 45/34* (2006.01)
*C07C 51/25* (2006.01)

(21) Anmeldenummer: **99123915.3**

(22) Anmeldetag: **01.12.1999**

(54) **Katalysator enthaltend eine Multimetalloxidmasse zur gasphasenkatalytischen Oxidation organischer Verbindungen**

Catalyst comprising a multimetal oxide mass for gas phase oxidation of organic compounds

Catalyseur comprenant une masse d'oxydes multimétalliques pour l'oxydation catalytique en phase gazeuse des composés organiques

(84) Benannte Vertragsstaaten:
**BE DE FR**

(30) Priorität: **03.12.1998 DE 19855913**

(43) Veröffentlichungstag der Anmeldung:
**07.06.2000 Patentblatt 2000/23**

(73) Patentinhaber: **BASF Aktiengesellschaft 67056 Ludwigshafen (DE)**

(72) Erfinder:
• **Heiko, Arnold, Dr.**
  **Da Chang District Nanjing 210048 (CH)**
• **Harth, Klaus, Dr.**
  **67317 Altleiningen (DE)**
• **Neumann, Hans-Peter, Dr.**
  **67067 Ludwigshafen (DE)**
• **Hammon, Ulrich, Dr.**
  **68165 Mannheim (DE)**
• **Felder, Raimund, Dr.**
  **67434 Neustadt (DE)**

(74) Vertreter: **Isenbruck, Günter et al Isenbruck, Bösl, Hörschler, Wichmann, Huhn Patentanwälte Theodor-Heuss-Anlage 12 68165 Mannheim (DE)**

(56) Entgegenhaltungen:
**DE-A- 2 431 511      FR-A- 2 364 061
US-A- 4 129 600**

**Beschreibung**

Die Erfindung betrifft einen Katalysator in Form von Splitt, Formkörpern oder eines Schalenkatalysators, der eine Multimetalloxidmasse als Aktivkomponente im wesentlichen der Formel

$$Mo_{12}Bi_aX^1_bFe_cX^2_dX^3_eO_y \qquad (I),$$

wobei

$X^1$ = Co und/oder Ni, bevorzugt Co,
$X^2$ = Si und/oder Al, bevorzugt Si,
$X^3$ = Alkali, vorzugsweise K, Na, Cs und/oder Rb, insbesondere K,
$0,3 \le a \le 1$,
$4 \le b \le 8$,
$2 \le c \le 4$,
$0 \le d \le 10$,
$0,05 \le e \le 0,2$ und
y dem Betrag der Zahl entspricht, der sich unter der Voraussetzung der Ladungsneutralität aus den Wertigkeiten und den stöchiometrischen Koeffizienten der übrigen Elemente ergibt, wobei in den kristallinen Anteilen neben $\beta$-(Co bzw. Ni)$MoO_4$ als Hauptkomponente $Fe_2(MoO_4)_3$ als Nebenkomponente und kein $MoO_3$ vorliegt, sowie als zusätzliche Nebenkomponenten $Bi_2Mo_3O_{12}$, $X^1_6Mo_{12}Fe_4Bi_{1,5}O_x$ oder Gemische davon in den kristallinen Anteilen, wobei x die für y angegebene Bedeutung hat und zusätzlich $Bi_2Mo_3O_{12}$ als weiteres Bismuthaltiges Oxid aufweist. Weiterhin betrifft die Erfindung Katalysators. .

Der Ausdruck "im wesentlichen" bedeutet vorzugsweise einen Gehalt von mindestens 95 Gew.-%, besonders bevorzugt mindestens 99 Gew.-%. Insbesondere liegen keine anderen Komponenten vor

Ähnliche Multimetalloxidmassen sind aus der EP-A 0 000 835 bekannt. Gemäß dieser Druckschrift werden diese Multimetalloxidmassen dadurch gebildet, daß das die katalytische Schlüsselphase dieser Multimetalloxidmassen bildende Bismut-Molybdän-Mischoxid in Abwesenheit der übrigen Konstituenten vorgebildet, nach seiner Vorbildung mit Quellen der übrigen Konstituenten der MultimetalloxidMassen vermischt und das Gemisch nach Trocknung calciniert wird.

Aus der EP-B 0 575 897 sind Multimetalloxidmassen bekannt, die dreidimensional ausgedehnte Bereiche der chemischen Zusammensetzung $Bi_2W_2O_9$ mit einem Größtdurchmesser von 1 bis 25 $\mu$m aufweisen. Diese Bereiche sind von ihrer lokalen Umgebung aufgrund ihrer chemischen Zusammensetzung abgegrenzt. Die bekannten Massen werden mittels eines Verfahrens gebildet, bei dem man zunächst ein feinteiliges Pulver aus calciniertem Mischoxid vorlegt und anschließend die Quellen der übrigen Bestandteile der Multimetalloxidmasse hinzu gibt.

Beide vorstehend genannten Multimetalloxidmassen werden zur gasphasenkatalytischen Oxidation organischer Verbindungen verwendet. Abgesehen von ihrer zeit- und arbeitsaufwendigen Herstellung sind die bekannten katalytischen Multimetalloxidmassen hinsichtlich ihrer Aktivität und auch ihrer Selektivität noch nicht vollkommen befriedigend.

In EP-A-0 493 274 und DE-A-27 41 132 sowie DE-A-31 14 709 ist die Herstellung von Multimetalloxidmassen durch Fällung beschrieben, wobei eine gemischte Metallsalzlösung der Ausgangsmetalle mit Ammoniakmolybdatlösung gefällt wird. Die Aktivität und Selektivität der erhaltenen Katalysatoren ist nicht für alle Anwendungen ausreichend.

Der Erfindung liegt dementsprechend die Aufgabe zugrunde, Katalysatoren enthaltend Multimetalloxidmassen bereitzustellen, die die vorstehend genannten Nachteile nicht aufweisen. Insbesondere sollen die Katalysatoren enthaltend Multimetalloxidmassen in bezug auf ihre Aktivität und Selektivität bei der Gasphasenoxidation verbessert sein und eine gesteigerte Raum-Zeit-Ausbeute erlauben.

Diese Aufgabe wird durch den eingangs aufgeführten Katalysator gelöst. Die Bestimmung Komponenten der Multimetalloxidmasse erfolgt dabei mittels Röntgendiffraktometrie.

Vorzugsweise liegt in den kristallinen Anteilen kein $Bi_3FeMo_2O_{12}$ vor.

$X^1$ kann Co und/oder Ni sein. Bevorzugt sind Gemische mit einem überwiegenden Anteil von Co, insbesondere ist $X^1$ = Co.

Die Herstellung derartiger Multimetalloxidmassen kann erfolgen, in dem man

a) eine erste wäßrige Lösung A der Konstituenten Bi, Fe und $X^1$ herstellt,
b) eine zweite wäßrige Lösung B der Konstituenten Mo und $X^3$ herstellt,
c) die Lösungen A und B vermischt, wobei eine Lösung oder Aufschlämmung des Konstituenten $X^2$ mit der Lösung A, B oder deren Gemisch vermischt wird,

d) das in c) erhaltene Gemisch oder Fällungsprodukt trocknet,

e) das in d) erhaltene Produkt calciniert.

Vorzugsweise werden dabei die wäßrigen Lösungen A und B gemischt, und die Mischung davon wird mit einer Lösung oder Aufschlämmung des Konstituenten $X^2$ vermischt.

Das Calcinieren erfolgt vorzugsweise bei einer Temperatur im Bereich von 450 bis 490°C, insbesondere bei einer Temperatur im Bereich von 460 bis 480°C, speziell bei einer Temperatur im Bereich von 465 bis 477°C.

Das Trocknen erfolgt vorzugsweise mittels Sprühtrocknung.

Erfindungsgemäß bevorzugt werden die Multimetalloxidmassen der Formel I mittels eines Eintopf-Verfahrens hergestellt. Dabei wird zunächst eine erste wäßrige Lösung mit den Konstituenten Bi, Fe, $X^1$ hergestellt. Die Herstellung der ersten wäßrigen Lösung erfolgt aus geeigneten wasserlöslichen Ausgangsverbindungen der Konstituenten Bi, Fe und $X^1$. Derartige wasserlösliche Verbindungen der genannten Konstituenten sind Sulfate, Halogenide, Acetate, Formiate, Nitrate, Carbonate etc. Von diesen wasserlöslichen Ausgangsverbindungen sind für die Herstellung der ersten wäßrigen Lösung die Nitrate bevorzugt. Um die erste wäßrige Lösung herzustellen, kann es notwendig sein, die Ausgangsverbindungen bei erhöhter Temperatur, insbesondere bei einer Temperatur zwischen 50°C und 70°C zu lösen.

Gleichermaßen wird eine zweite wäßrige Lösung B der Konstituenten Mo und $X^3$ hergestellt. Insbesondere wird hierzu Ammoniummolybdat eingesetzt. Das Alkalimetall $X^3$ ist vorzugsweise Kalium, das als KOH eingesetzt wird. Auch diese Lösung kann bei einer Temperatur zwischen 50°C und 70°C hergestellt werden. Sodann werden die beiden Lösungen vermischt, beispielsweise, indem Lösung A in Lösung B gepumpt wird. Der Konstituent $X^2$ kann dabei in der Lösung A, der Lösung B oder dem Gemisch enthalten sein beziehungsweise diesen in Form einer Lösung oder Aufschlämmung zugesetzt sein. Vorzugsweise wird ein Kieselsol dem Gemisch zugesetzt, sofern in dem Gemisch $X^2$ Silicium ist.

Das Vermischen der Lösungen bewirkt eine Fällung der Metalle.

Falls die katalytische Multimetalloxidmasse Poren aufweisen soll, so ist es bevorzugt, als Einsatzstoffe Ammoniumsalze bzw. Nitrate der Ausgangsverbindungen einzusetzen, so daß ein porenhaltiger Katalysator entsteht. Werden keine Ammoniumsalze oder Nitrate eingesetzt, so kann man auch einer der beiden wäßrigen Lösungen oder auch beiden wäßrigen Lösungen Ammoniumnitrat als Porenbildner hinzuzufügen. Bei Bedarf kann einer oder beiden vorstehend genannten wäßrigen Lösungen auch eine feinteilige Suspension von Aluminiumoxid- und/oder Siliciumoxid-Teilchen hinzugefügt werden. Das Aluminiumoxid bzw. das Siliciumoxid fungiert als inerter Verdünner.

Nach erfolgter Fällung wird der Niederschlag von dem Rest des Lösungsmittels abgetrennt. Dazu kann er durch Eindampfen getrocknet werden. Bevorzugt erfolgt jedoch das Abtrennen mittels Sprühtrocknung. Die Sprühtrocknung kann unter Gleich- oder Gegenstromführung erfolgen. Während der Sprühtrocknung liegt die Temperatur am Sprüheintritt vorzugsweise bei ca. 400°C, die Temperatur am Sprühturmausgang bei ca. 120 bis 140°C. Durch das Sprühtrocknen werden Teilchen bzw. Partikel der erfindungsgemäßen Multimetalloxidmasse mit einem Durchmesser von etwa 1 bis 100 $\mu$m erhalten. Das nach dem Sprühtrocknen erhaltene Pulver wird je nach gewünschter Katalysatorform weiter verarbeitet. Zur Herstellung von Ringkatalysatoren wird das erhaltene Pulver unter Verwendung von maximal 4 Gew.-% Graphit (BET-Oberfläche 5-15 $m^2$/g, mittlerer Teilchendurchmesser < 30 $\mu$m) als Tablettierhilfsmittel tablettiert und sodann zur Oxidbildung bei einer Temperatur von vorzugsweise 150° bis 300°C zersetzt. Anschließend wird die resultierende oxidische Masse bei den vorstehend angegebenen Temperaturen calciniert. Der Verfahrensschritt der Zersetzung und derjenige der Calcinierung kann in einer Inertgasatmosphäre, an Luft, unter Sauerstoff, Stickstoff usw. erfolgen. Das Calcinieren und das Zersetzen können als Verfahrensschritte ineinander übergehen. Zur Herstellung von Splitt-Katalysatoren wird das nach dem Sprühtrocknen erhaltene Pulver geknetet, sodann getrocknet und grob zermahlen. Anschließend wird es wie beschrieben zersetzt und calciniert, woran sich ein Versplitten und Aussieben anschließen.

Zur Herstellung von Schalenkatalysatoren wird das nach der Sprühtrocknung erhaltene Pulver geknetet, getrocknet und grob zermahlen und sodann wie beschrieben zersetzt. Nach dem Zersetzen wird auf eine definierte Korngröße gemahlen, der Katalysatorträger beschichtet und erst anschließend calciniert.

Die Multimetalloxidmasse der Formel I $Mo_{12}Bi_aX^1_bFe_cX^2_dX^3_cO_y$ enthält als $X^1$ Co und/oder Ni, bevorzugt jedoch Co $X^2$ ist Si und/oder Al, bevorzugt Si. Bei $X^3$ handelt es sich um Alkalielemente Li, Na, K, Cs und/oder Rb vorzugsweise K, Na und/oder Cs, stärker bevorzugt jedoch um K; der Wert für die Variable a liegt im Bereich von 0,3 bis 1. Bevorzugt ist 0,4 ≤ a ≤ 1, insbesondere 0,4 ≤ a ≤ 0,95. Der Wert für die Variable b liegt im Bereich von 4 ≤ b ≤ 8, bevorzugt 6 ≤ b ≤8; der Wert für die Variable c liegt im Bereich von 2 bis 4. Der Wert für die Variable e ist 0,05 ≤ e ≤ 0,2.

Der Wert für O (Sauerstoff) ergibt sich aus dem Betrag der Zahl der Summe der Wertigkeiten bzw der stöchiometrischen Koeffizienten der übrigen Elemente, d.h. der Kationen. Bei dem erfindungsgemäßen Verfahren wird bevorzugt eine Multimetalloxidmasse hergestellt, bei der das Verhältnis von Co/Ni wenigstens 2:1, stärker bevorzugt wenigstens 3:1, noch stärker bevorzugt wenigstens 4:1 beträgt. Am besten liegt nur Co vor. Die mittels des erfindungsgemäßen Verfahrens erhältliche Multimetalloxidmasse zeichnet sich neben der vorstehend beschriebenen Gesamtzusammensetzung ihrer Bestandteile durch eine charakteristische Phasenzusammensetzung aus, wie sie in den Beispielen anhand der Röntgendiffraktogramme nachgewiesen wird.

Bei besonders bevorzugten erfindungsgemäßen Multimetalloxidmassen liegt der Wert für 1,5 (a + c) + b zwischen 11 und 14, vorzugsweise zwischen 11,5 und 13.

Besonders bevorzugt sind Werte für 1,5 (a + c) + b im Bereich zwischen 11,8 und 12,5. Die Grenzwerte sind in den genannten Bereichsangaben miteingeschlossen.

Die erfindungsgemäße Multimetalloxidmasse wird erfindungsgemäß zur Herstellung von Formkörpern, insbesondere von Katalysator-Formkörpern verwendet. Dazu wird sie als Pulver eingesetzt. Die Formgebung selber kann vor oder nach der Kalzinierung erfolgen. Als Formkörper sind Voll- oder Schalenkatalysatoren möglich. Bevorzugt sind aus der erfindungsgemäßen Multnnetalloxidmasse hergestellte Schalenkatalysatoren oder ringförmige Vollkatalysatoren, die durch Tablettieren oder Extrudieren erhalten werden können Zudem kann der Katalysator als Splitt vorliegen.

Der Katalysator enthält neben den erfindungsgemäßen Multimetalloxiden noch ein Bismut-haltiges Oxid $Bi_2Mo_3O_{12}$. Die Oxide können dabei als Pulver vermischt und zu den erfindungsgemäßen Katalysatoren verarbeitet werden.

Die erfindungsgemäßen Katalysatoren eignen sich für die selektive Gasphasenoxidation von organischen Verbindungen, insbesondere Alkenen wie Propen. Insbesondere eignen sie sich für die Herstellung von $\alpha,\beta$-ungesättigten Aldehyden und/oder Carbonsäuren aus Alkenen, Alkanen, Alkanonen oder Alkenalen. Besonders bevorzugt werden die erfindungsgemäßen Katalysatoren zur Herstellung von Acrolein und Acrylsäure aus Propen bzw. Memacrolein und Methacrylsäure aus i-Buten eingesetzt.

Die Erfindung wird durch die nachfolgenden Beispiele näher erläutert:

**BEISPIELE**

**1. Herstellung der Aktivkomponente (Multimetalloxidmasse) (Ansatzgröße ca. 4-5 kg Sprühpulver) Vorgehensweise für $Bi_{1,0}$-Ansatz:**

Es werden 2 Lösungen hergestellt:

a) Eisen-Cobalt-Bismut-Nitrat Lösung (Lösung A)

Zu 3427,6 g einer auf 60°C vorgeheizten Cobaltnitratlösung (12,4 Gew.-% Co) gibt man unter Rühren 1191,4 g Eisennitrat (14,2 Gew.-% Fe). Nach Beendigung der Zugabe wird noch 30 min nachgerührt, wobei die Temperatur bei 60°C konstant gehalten wird. Zuletzt erfolgt die Zugabe von 1922,5 g der Bismutnitrat-Lösung (11,2 Gew.-% Bi). Bei einer Temperatur von 60°C wird nochmals 10 min nachgerührt.

b) Molybdän-Kalium-Lösung (Lösung B)

Zu 2500 g VE-Wasser gibt man unter Rühren 9,88 g KOH-Lösung (46,8 Gew.-% KOH) und heizt diese unter Rühren auf 60°C auf. In dieser Lösung werden bei 60°C unter Rühren 2182,9 g Ammoniumheptamolybdat gelöst. Man rührt diese Lösung 1 Stunde, wobei eine klare Lösung erhalten wird.

**Fällen:** Zu der vorgelegten Lösung B gibt man über eine Pumpe unter Rühren Lösung A innerhalb von 15 min zu. Nach Beendigung der Zugabe wird noch mindestens 5 min nachgerührt, anschließend erfolgt die Zugabe von 188,2 g Kieselsol (50 Gew.-% $SiO_2$, Dichte 1,36 bis 1,42 g/ml, pH 8,5 bis 9,5, Alkaligehalt max. 0,5 Gew.-%). Es wird nochmals für mindestens 5 min nachgerührt. Während dieser Zeit wird die Temperatur des Fällansatzes bei 60°C konstant gehalten.

**Sprühtrocknen:** Diese Lösung wird dann in einem Sprühtrockner versprüht. Die Eintrittstemperatur beträgt hierbei 380 +/- 10°C, die Austrittstemperatur 115 +/- 5°C. Die chemische Zusammensetzung des so erhaltenen Pulvers (4266 g), im folgenden SPLV genannt, beträgt $Mo_{12}Co_7Fe_{2,94}Bi_1Si_{1,52}K_{0,08}O_x$.

Als weitere Beispiele sind mit wechselnden Bi-Gehalten folgende SPLV präpariert worden:

**Tabelle 1: Zugabe Bismut-Lösung für Fällansatz**

| Br-Gehalt (bez $Mo_{12}$) | Zugabe Bi-Lsg. [g] |
| --- | --- |
| 0 (Vergl.) | |
| 0,3 | 576,7 |
| 0,45 | 865,1 |
| 0,6 | 1153,5 |
| 0,8 | 1538 |

(fortgesetzt)

| Br-Gehalt (bez Mo$_{12}$) | Zugabe Bi-Lsg. [g] |
|---|---|
| 1 | siehe oben |
| 1,5 (Vergl.) | 2884 |
| 2 (Vergl.) | 3845 |
| 3(Vergl.) | 5767 |

**2. Herstellung und Testung der Katalysatoren aus Multimetalloxidmasse**

**2.1 Herstellung und Testung von Katalysatorsplit+Eintopf-Sprühansatz (Vergleichversuche)**

a) **Kneten:** 400 g des Sprühpulvers der Zusammensetzung Mo$_{12}$Co$_7$Fe$_y$-Si$_{1,5}$K$_{0,08}$O$_x$(y=0 bis 3) werden in einem 21 Kneter nach der Zugabe von 45 bis 65 ml Wasser geknetet. Die Knetung erfolgt in 3 Schritten von 5, 10 und 15 min. Nach 5 beziehungsweise 10 min Knetzeit wird das Knetgut nochmals von Hand zerteilt und durchmischt, um eine gleichmäßige Einarbeitung zu gewährleisten.

b) **Trocknung:** Im Anschluß an die Knetung erfolgt die Trocknung des grob zerteilten Knetguts während 2 h in einem Trockenschrank bei 120°C. Das so getrocknete Knetgut wird durch mechanische Beanspruchung und fraktioniertes Sieben auf eine Korngröße von 0,71 bis 1,6 mm gebracht (Splitten).

c) **Zersetzung:** Die Zersetzung des zerkleinerten Katalysatorsplitts erfolgt in einem Labordrehrohr während 2 h bei einer Temperatur von 250°C im Stickstoffstrom.

d) **Calcination:** Die Calcination erfolgt ebenfalls in einem Labordrehrohr während 6 h bei einer Temperatur von 466 bis 471°C im Luftstrom.

Der so erhaltene Katalysatorsplitt wird wie folgt getestet:
Einbau von ca. 30 g dieses Splitts in einen Rohrreaktor mit einem lichten Durchmesser von 8 mm. Dabei werden üblicherweise Schutthöhen von ca. 65 cm im Rohrreaktor erreicht (entspr. einer Schüttdichte von 0,9 bis 0,95 g/ml). Bei einer konstanten Betriebstemperatur von 359°C wird die Menge an Feedgas (Gemisch aus 5 Vol.-% Propen, 9,5 Vol.-% Sauerstoff und 85,5 Vol.-% Stickstoff) solange variiert, bis der Propen-Umsatz gemessen als

$$U_{Propen}[\%] = \frac{C_{Propen,Eingang} - C_{Propen,Ausgang}}{C_{Propen,Eingang}} \cdot 100$$

95% beträgt. Gemessen wird die Selektivität zu den Wertprodukten Acrolein (S$_{ACR}$) und Acrylsäure (S$_{ACS}$). Diese berechnen sich wie folgt:

$$S_{ACR}[\%] = \frac{C_{ACR,Ausgang}}{C_{Propen,Eingang} - C_{Propen,Ausgang}} \cdot 100$$

$$S_{ACS}[\%] = \frac{C_{ACS,Ausgang}}{C_{Propen,Eingang} - C_{Propen,Ausgang}} \cdot 100$$

Die Selektivität für die Wertprodukte (Swp) beträgt:

$$S_{WP}[\%] = S_{ACS} + S_{ACR}$$

Bei der Testung dieser Vergleichs-Katalysatoren wurden folgende Werte bestimmt:

**Tabelle 2: Performance des Vergleichs-Katalysstorsplitts enthaltend in Multimetalloxidmasse**

| Lfd.Nr. | $T_{Calc.}$ [°C]/6 h | Bi-Gehalt | Belastung[1] | $U_{Propen}$ [%] | $S_{ACR}$ [%] | $S_{ACS}$ [%] | $S_{WP}$ [%] |
|---------|----------------------|-----------|--------------|------------------|---------------|---------------|--------------|
| V1 | 467 | 0 | 1,74 | 60 | 74,5 | 0 | 74,5 |
| 2 | 471 | 0,3 | 3 | 95 | 90,3 | 6,8 | 97,1 |
| 3 | 469 | 0,6 | 4,9 | 95 | 90,2 | 7,4 | 97,6 |
| 4 | 471 | 1 | 3 | 95 | 89,6 | 4,1 | 93,7 |
| [1] ausgedrückt als $Nl_{Propen}$/h | | | | | | | |

### 2.2 Herstellung und Testung von Vergleichs-Schalenkatalysatoren, Eintopf-Sprühansatz

Bei der Herstellung der Schalenkatalysatoren verfährt man bei den verfahrensschritten Kneten, Trocknen und Zersetzung wie unter 2.1 beschrieben Daran schließen sich folgende Schritte an:

a) **Mahlung:** In einer Zentrifugalmühle (Typ ZM 100, Retsch) wird der zersetzte Katalysatorvorläufer auf eine Korngröße ≤ 0,12 mm gemahlen.

b) **Beschichtung:** Dieses Pulver wird in einer an sich bekannten Art auf Steatitkugeln (nicht porös, Durchmesser 3 bis 4 mm) aufgebracht (siehe z.B. DE-A 29 09 671 und EP-A 0 293 859). Beschichtungshilfsmittel ist in der Regel Wasser oder ein Wasser/Glycerin-Gemisch, kann aber auch, z.B. zur Erlangung besonders abriebfester Katalysatoren, ein Gemisch aus Wasser/Alkohol und Tertramethoxysilan beziehungsweise Tetraethoxysilan sein. Als Alkohole kommen Methanol, Ethanol, Isopropanol, n-Butanol und tert.-Butanol in Frage. Der Schalenanteil (S) berechnet sich wie folgt:

$$S[\%] = 100 - \frac{m_{Steatit}}{m_{Steatit} + m_{Aktivkomponente}} \bullet 100$$

c) **Calcination:** wie unter 2.1 beschrieben, Temperatur siehe Tabelle 3.

Bei diesen Schalenkatalysatoren erfolgt die Testung ebenfalls in einem Reaktor mit einem lichten Durchmesser von 15 mm. Eingefüllt werden hier 120 g Katalysator Die Reaktionstemperatur wird hierbei solange variiert, bis der Propen-Umsatz bei einer Propenladung von 5 $Nl_{Propen}$/h 95 % erreicht hat.

**Tabelle 3: Performance der Vergleichs-Schalenkatalysatoren**

| Lfd.Nr. | Bi-Anteil | $T_{Cal.}$ | S | Belastung | Temperatur | $U_{Propen}$ | $S_{ACR}$ | $S_{ACS}$ | $S_{WP}$ |
|---------|-----------|------------|---|-----------|------------|--------------|-----------|-----------|----------|
| 5 | 0,3 | 477 | 50 | 4,9 | 386 | 95,5 | 73,4 | 21,8 | 95,2 |
| 6 | 0,45 | 474 | 51 | 5 | 304 | 95 | 89,5 | 3,9 | 93,4 |
| 7 | 0,6 | 468 | 50 | 5 | 321 | 95,1 | 90,8 | 5,6 | 96,4 |
| 8 | 0,8 | 474 | 50 | 5 | 318 | 95 | 91,6 | 4,3 | 95,9 |
| v9 | 2 | 466 | 50 | 4,7 | 311 | 95,5 | 72,8 | 3,5 | 76,3 |
| v10 | 3 | 473 | 52 | 5 | 313 | 95,2 | 86,2 | 3,4 | 89,6 |

### 2.3 Herstellung und Testung von erfindungsgemäßen Katalysatoren aus getrennt präparierten Pulvern

Im folgenden werden erfindungsgemäße Katalysatoren beschrieben, die durch Mischen zweier getrennt präparierter Pulver hergestellt wurden. Die Präparation der erfindungsgemäßen Multimetalloxid-pulver 1 entspricht der oben beschriebenen Vorgehensweise. Vor dem Kneten der Aktivmasse erfolgt eine homogene Durchmischung der beiden Pulver, die in den in der Tabelle angegebene Gewichtsverhältnissen gemischt werden. Es wird ein zusätzliches Katalysatorpulver (im folgenden Pulver 2 genannt) verwendet:

1. $Bi_2Mo_3O_{12}$ (ASTM-Nr. 21-0103)

In 500 g VE-Wasser werden bei 60°C 389,2 g Ammoniumheptamolybdat gelöst. Zu dieser Lösung gibt man unter Rühren im Verlauf von 90 min eine 11,2 Gew.-% Bismut enthaltende Bi-Nitrat-Lösung. Nach beendeter Zugabe wird die Suspension noch 30 min gerührt. Mittels eines Sprühtrockners wird die Suspension getrocknet. Das getrocknete Pulver wird in einem Laborofen bei einer Temperatur von 220°C während 2 h zersetzt. Die Calcination erfolgt bei einer Temperatur von 580°C während 8 h. Die so erhaltene Substanz kann durch ein Röntgendiffraktogramm charakterisiert werden. Diese Substanz besitzt die typischen Reflexe, wie sie für diese Verbindung in der ASTM-Kartei unter der Nummer 21-0103 zu finden sind.

Nachfolgende Tabelle stellt alle nach diesem Konzept präparierten Katalysatoren in der Übersicht dar. Das sogenannte Pulver 1 entspricht den weiter oben genannten Beispielen.

Die Testung erfolgt entsprechend der weiter oben beschriebenen Vorgehensweise, d.h. Splitt wird im 8 mm Rohr bei 358°C und die Schalenkatalysatoren werden bei einer festen Propen-Beladung von 5 $Nl_{Propen}$/h in einem 15 mm Rohr getestet.

**Tabelle 4: Präparation gemischtphasiger erfindungsgemäße Katalysatoren**

| Lfd.NR. | Bi-Gehalt Pulver 1 (entspr.Lfd.Nr.) | Pulver 2 | Mischungsverhältnis [gPlv.1/g-Plv.2] | Schalenanteil | $T_{Calc.}$[°C]/6h |
|---------|-------------------------------------|----------|--------------------------------------|---------------|---------------------|
| 11 | 0,6 (Nr.3) | $Bi_2Mo_3O_{12}$ | 500/52 | 50 | 471 |
| 12 | 1,0 (Nr.4) | $Bi_2Mo_3O_{12}$ | 550/55,2 | 51 | 473 |
| 13 | 0,6 (Nr.3) | $Bi_2Mo_3O_{12}$ | 400/41,7 | Splitt | 469 |
| 16 | 0,3 (Nr.2) | $Bi_2Mo_3O_{12}$ | 400/42,7 | Splitt | 478 |

**Tabelle 5: Performance der gemischtphasigen erfindungsgemäße Katalysatoren**

| Lfd.Nr. | Bi-Anteil (Summe) | Belastung[1] | Temp. | $U_{Propen}$ | $S_{ACR}$ | $S_{ACS}$ | $S_{WP}$ |
|---------|-------------------|--------------|-------|--------------|-----------|-----------|----------|
| 11 | 1,6 | 4,9 | 345v | 94,7 | 87,3 | 9,7 | 97 |
| 12 | 2 | 5,2 | 325 | 95,0 | 91,4 | 4,4 | 95,8 |
| 13 | 1,6 | 2,45 | 359 | 95,0 | 88 | 9,4 | 97,4 |
| 16 | 1,3 | 3,35 | 358 | 95,0 | 90,1 | 6,9 | 97 |

### 3. Charakterisierung der Multimetalloxidmassen

### 3.1 Porosimetrie (Splitt)

Porenvolumen (aus Quecksilber-Porosimetrie)0,3-0,35 ml/g
BET-Oberfläche (aus $N_2$-Sorption)5-8 $m^2$/g

### 3.2 Analysen zu den Schalenkstalysatoren

Porenvolumen (aus Quecksilber-Porosimetrie)0,2-0,3 ml/g
BET-Oberfläche (aus $N_2$-Sorption)7-13 $m^2$/g

### 3.3 Phasenzusammensetzung

In dem Multimetalloxidmassen können mittels Röntgendiffraktometrie folgende Festkörperphasen nachgewiesen werden:

**Tabelle 8: Im Röntgendiffraktogramm charakterisierbare Phasen in Abhängigkeit von der Sprühpulverzusammensetzung**

| Bi-Gehalt | B-Co-MoO$_4$ 210868 | Fe$_2$(MoO$_4$)$_3$ 310642 | MoO$_3$ 5-0508 | β-Co-MoO$_4$ 251434 | Bi$_2$Mo$_3$O$_{12}$ 210103 | Co$_6$Mo$_{12}$Fe$_4$Bi$_{1,5}$O$_x$ 370974 | Bi$_3$Fe-Mo$_2$O$_{12}$ 270047 |
|---|---|---|---|---|---|---|---|
| 0 | + | + | + | + | - | - | - |
| 0,3 | + | + | - | + | + | - | - |
| 0,45 | + | + | - | - | - | + | - |
| 0,6 | + | + | - | + | + | + | - |
| 0.8 | + | + | - | - | - | + | - |
| 1 | + | + | - | + | + | + | - |
| 2 | + | + | - | - | - | - | + |
| 3 | + | + | - | - | - | - | + |

Die zweite Zeile gibt in der Tabelle die ASTM-Nummem an.

Es ergibt sich folgendes:

1. β-CoMoO$_4$ als Hauptkomponente des Katalysators. Fe$_2$(MoO$_4$)$_3$ als wichtigste Nebenkomponente.

2. Ein Ausschluß des MoO$_3$ sobald Bi zugegeben worden ist.

3. Eine Bildung der Verbindungen Bi$_2$Mo$_3$O$_{12}$ beziehungsweise Co$_6$Mo$_{12}$Fe$_4$Bi$_{1,5}$O$_x$ im Bereich (Mo$_{12}$Bi$_{0,3}$ bis Mo$_{12}$Bi$_{1,0}$) der Bi-Zugabe (eine exaktere Differenzierung zwischen den beiden Verbindungen ist infolge vielfältigen Linienüberlagerungen mit anderen Phasen schwer möglich).

4. Eine Bildung einer unerwünschten, da unselektiven Phase (Bi$_3$FeMo$_2$O$_{12}$) bei einer höheren Bi-Zugabe (Mo$_{12}$Bi$_2$ und Mo$_{12}$Bi$_3$).

**Patentansprüche**

1. Katalysator in Form von Splitt, Formkörpern oder eines Schalenkatalysators, der eine Multimetalloxidmasse, als Nativkomponente im wesentlichen bestehend aus

$$Mo_{12}Bi_aX^1_bFe_cX^2_dX^3_eO_y \qquad (I)$$

wobei:

X$^1$ = Co und/oder Ni,
X$^2$ = Si und/oder Al,
X$^3$ = Alkali,
0,3 ≤ a ≤ 1,
4 ≤ b ≤ 8,
2,5 ≤ c ≤ 14,
0 ≤ d ≤ 10,
0,05 ≤ e ≤ 0,2 und
y dem Betrag der Zahl entspricht, der sich unter der Voraussetzung der Ladungsneutralität aus der Summe der Wertigkeiten und der stöchiometrischen Koeffizienten der übrigen Elemente ergibt, wobei in den kristallinen Anteilen neben β-X$^1$MoO$_4$ als Hauptkomponente Fe$_2$(MoO$_4$)$_3$ als Nebenkomponente und kein MoO$_3$ vorliegt, sowie als zusätzliche Nebenkomponenten Bi$_2$Mo$_3$O$_{12}$, X$^1_6$Mo$_{12}$Fe$_4$Bi$_{1,5}$O$_x$ oder Gemische davon in den kristallinen Anteilen vorliegen, wobei x die für y angegebene Bedeutung hat, und zusätzlich Bi$_2$Mo$_3$O$_{12}$ als weiteres Bismuthaltiges Oxid aufweist.

2. Verwendung eines Katalysators nach Anspruch 1 zur selektiven Gasphasenoxidation von Alkenen

**3.** Verwendung nach Anspruch 2 zur Herstellung von α,β-ungesättigten Aldehyden und/oder Carbonsäuren.

**Claims**

**1.** A catalyst in the form of chips or moldings or a coated catalyst comprising a multimetal oxide material as active component essentially consisting of

$$Mo_{12}Bi_aX^1{}_bFe_cX^2{}_dX^3{}_eO_y \qquad (I)$$

where:

$X^1$ is Co and/or Ni,
$X^2$ is Si and/or Al,
$X^3$ is an alkali metal,
$0.3 \leq a \leq 1$,
$4 \leq b \leq 8$,
$2 \leq c \leq 4$,
$0 \leq d \leq 10$,
$0.05 \leq e \leq 0.2$ and
y is the absolute value of the number which is obtained, assuming charge neutrality, from the sum of the valences and the stoichiometric coefficients of the other elements, the crystalline fractions containing, in addition to β-$X^1MoO_4$ as the main component, $Fe_2 (MoO_4)_3$ as a secondary component and no $MoO_3$ and containing $Bi_2Mo_3O_{12}$, $X^1{}_6Mo_{12}Fe_4Bi_{1.5}O_x$ or a mixture thereof as an additional secondary component in the crystalline fractions, x having the meaning stated for y, and additionally having $Bi_2Mo_3O_{12}$ as further bismuth-containing oxide.

**2.** The use of a catalyst according to claim 1 for the selective gas-phase oxidation of alkenes.

**3.** The use according to claim 2 for the preparation of α,β-unsaturated aldehydes and/or carboxylic acids.

**Revendications**

**1.** Catalyseur sous forme de gravillon, de corps façonnés ou d'un catalyseur en coques, qui présente une masse d'oxyde multimétallique modifiée comme composant actif, qui est essentiellement constituée de

$$Mo_{12}Bi_aX^1{}_bFe_cX^2{}_dW^3{}_eO_y \qquad (I)$$

où

$X^1$ = Co et/ou Ni,
$X^2$ = Si et/ou A1,
$X^3$ = alcali,
$0,3 \leq a \leq 1$,
$4 \leq b \leq 8$,
$2 \leq c \leq 4$,
$0 \leq d \leq 10$,
$0,05 \leq e \leq 0,2$ et
y correspond à la valeur du chiffre qui, en présupposant la neutralité de charge, résulte de la somme des valences et des coefficients stoechiométriques des autres éléments, dans laquelle il y a, dans les fractions cristallines, à côté de β-$X^1MoO_4$ comme composant principal, $Fe_2(MoO_4)_3$ comme composant secondaire et aucun $MoO_3$, et dans laquelle il y a, dans les fractions cristallines, comme composants secondaires additionnels, $Bi_2Mo_3O_{12}$, $X^1{}_6Mo_{12}Fe_4Bi_{1,5}O_x$ ou leurs mélanges, x ayant la signification indiquée pour y, et qui présente en supplément $Bi_2Mo_3O_{12}$ comme autre oxyde contenant du bismuth.

**2.** Utilisation d'un catalyseur suivant la revendication 1, pour l'oxydation en phase gazeuse sélective d'alcènes.

**3.** Utilisation suivant la revendication 2, pour la préparation d'acides carboxyliques et/ou aldéhydes $\alpha,\beta$-insaturés.